# EUROPÄISCHE PATENTANMELDUNG

(11) **EP 0 815 800 A1**
(43) Veröffentlichungstag der Anmeldung: **07.01.1998**
(21) Anmeldenummer: 97110768.5
(22) Anmeldetag: 01.07.1997
(51) Int. Cl.: A61B 17/92, A61B 17/068

(54) **Setzwerkzeug für Nägel oder dergleichen**

(30) Priorität: 03.07.1996 DE 19626892
(71) Anmelder: Kirsch, Axel, Dr., 70772 Filderstadt (DE)
(72) Erfinder: Kirsch, Axel, D-70772 Filderstadt (DE); Dürr, Walter, D-79196 Remchingen (DE)
(74) Vertreter: Goddar, Heinz J., Dr.

(57) **Zusammenfassung**

Ein Setzwerkzeug für Nägel, mit einer an beiden Enden offenen hohlzylindrischen Griffhülse, bei der ein erster Hohlabschnitt mit einem zweiten Hohlabschnitt durch eine Durchgangsöffnung verbunden ist, in die folgende Komponenten eingelegt sind: ein Stößel, an dem ein Nagel direkt oder indirekt lösbar und aus der Griffhülse freiliegend zu halten ist, ein Bolzen, der von einer Schraubenfeder umgeben ist, deren Achse aus der Achse der Griffhülse versetzt ist, und der mit dem Anlageabschnitt eines ersten Bolzenabschnitts an dem Stößel anliegt und bei dem im Übergangsbereich von dem ersten Bolzenabschnitt zu einem zweiten Bolzenabschnitt eine Zentrierfläche vorgesehen ist, ein zylindrisches Kopplungsstück, das an seiner dem Bolzen zugewandten Seite mit einem Blindloch versehen ist, in das der zweite Bolzenabschnitt des Bolzens zumindest teilweise eintauchen kann und dessen Durchmesser kleiner ist als der Durchmesser der Durchgangsöffnung, eine zweite Schraubenfeder, eine Einstellvorrichtung, die verstellbar in das zweite offene Ende der Griffhülse eingesetzt ist, wobei die zweite Schraubenfeder einerseits an dem Kopplungsstück und andererseits an der Einstellvorrichtung anliegt und wobei der Stößel und der Bolzen mit der ihn umgebenden Schraubenfeder zumindest teilweise im ersten Hohlabschnitt und das Kopplungsstück die zweite Schraubenfeder und die Einstellvorrichtung im zweiten Hohlabschnitt untergebracht sind.

## Beschreibung

Die Erfindung betrifft ein Setzwerkzeug für Nägel oder dergleichen, beispielsweise eines Befestigungsnagels für körpereigenen Knochen.

Ein aus der DE 43 00 039 C1 bekanntes Setzwerkzeug besteht aus einem langgestreckten, bleistiftartigen Griffteil, an dessen einem Ende ein Druck-Schlag-Kopf und an dessem anderen Ende eine mit mindestens zwei federnden Greifbacken versehene Halteeinrichtung für einen Befestigungsnagel angeordnet ist. Der Operateur ergreift das Setzwerkzeug mit der Hand, woraufhin der Befestigungsnagel in den Knochen eingedrückt oder unter Zuhilfenahme eines den Druck-Schlag-Kopf beaufschlagenden Hammers in den Knochen eingetrieben wird.

Es ist die Aufgabe der vorliegenden Erfindung, ein Setzwerkzeug für Nägel oder dergleichen bereitzustellen, bei dem auf das Beaufschlagen mit einem Hammer verzichtet werden kann.

Diese Aufgabe wird von einem Setzwerkzeug nach Anspruch 1 gelöst. Vorteilhafte Ausgestaltungen sind Gegenstand der Unteransprüche.

Erfindungsgemäß besteht die Setzeinrichtung für Nägel aus einer an beiden Enden offenen hohlzylindrischen Griffhülse, bei der ein erster Hohlabschnitt mit einem zweiten Hohlabschnitt durch eine Durchgangsöffnung verbunden ist, in die folgende Komponenten eingelegt sind: ein Stößel, an dem ein Nagel direkt oder indirekt lösbar und aus der Griffhülse freiliegend zu halten ist, ein Bolzen, der von einer Schraubenfeder umgeben ist, deren Achse aus der Achse der Griffhülse versetzt ist, und der mit dem Anlageabschnitt eines ersten Bolzenabschnittes an dem Stößel anliegt und bei dem im Übergangsbereich von dem ersten Bolzenabschnitt zu einem zweiten Bolzenabschnitt eine Zentrierfläche vorgesehen ist, ein Kopplungsstück, das an seiner dem Bolzen zugewandten Seite mit einem Blindloch versehen ist, in das der zweite Bolzenabschnitt zumindest teilweise eintauchen kann, und dessen Durchmesser kleiner ist als der Durchmesser der Durchgangsöffnung, eine zweite Schraubenfeder und eine Einstellvorrichtung, die verstellbar in das zweite offene Ende der Griffhülse eingesetzt ist, wobei die zweite Schraubenfeder einerseits an dem Kopplungsstück und andererseits an der Einstellvorrichtung anliegt, und wobei der Stößel und der Bolzen mit der ihn umgebenden Schraubenfeder zumindest teilweise im ersten Hohlabschnitt und das Kopplungsstück, die zweite Schraubenfeder und die Einstellvorrichtung in dem zweiten Hohlabschnitt untergebracht sind.

Vorteilhaft sind der Anlageabschnitt des Bolzens an den Stößel und/oder der Anlageabschnitt des Stößels an den Bolzen an ihren Kontaktbereich gewölbt ausgebildet. Bei der Erfindung kann das freie Ende des Stößels als Halterung für den Kopf eines Nagels ausgebildet sein. Ein geeigneter Haltemechanismus ist in der DE 43 00 039 C1 beschrieben.

In einer Ausgestaltung der Erfindung sind der Stößel und der Bolzen hohlzylindrisch ausgebildet, wobei in dem Bolzen ein Innenbolzen untergebracht ist, dessen Länge größer ist als die Länge des Bolzens, und der direkt oder indirekt auf einen Nagelhalter wirkt, welcher in den Stößel eingesetzt ist.

Weiter bevorzugt ist in dem hohlzylindrischen Stößel ein Kopplungsbolzen untergebracht.

Der Stößel kann weiterhin Sichtfenster aufweisen, die den Blick auf den Nagelhalter freigeben.

Es ist besonders bevorzugt, wenn der Stößel eine Rasteinrichtung zum Eingriff mit dem Nagelhalter aufweist.

Zweckmäßigerweise kann die Einstellvorrichtung eine Einstellschraube sein, deren Gewindeabschnitt in ein in der Griffhülse vorgesehenes Innengewinde eingreift.

Für dieses federbetriebene Setzwerkzeug ist ein Hammer oder ähnliches Schlagwerkzeug nicht mehr vonnöten. Die Schlagkraft wird durch die zweite Schraubenfeder festgelegt, eine Veränderung der Schlagkraft wird dadurch hervorgerufen, daß die Einstellvorrichtung die Feder mehr oder weniger vorspannt.

Im folgenden soll die Erfindung anhand der beigefügten Zeichnung näher erläutert werden.

Dabei zeigt
- Figur 1: in einer kombinierten Seitenansicht/Längsschnittansicht ein Beispiel für eine Griffhülse eines Setzwerkzeuges gemäß der vorliegenden Erfindung;
- Figur 2: die Draufsicht auf die Griffhülse von hinten;
- Figur 3: eine erste Ausführungsform des Schlagfedermechanismus für das Setzwerkzeug gemäß der vorliegenden Erfindung;
- Figur 4: eine zweite Ausführungsform des Schlagfedermechanismus; und
- Figur 5: ein Beispiel für einen Nagelhalter.

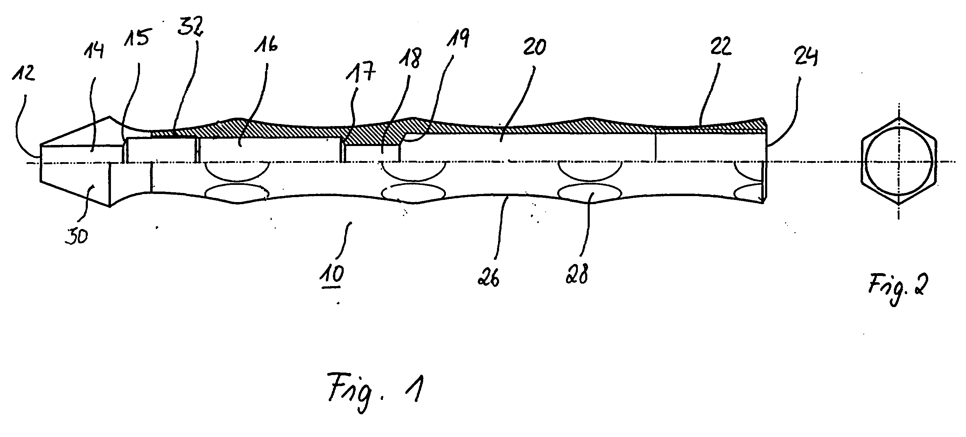

Die in Figur 1 dargestellte Griffhülse ist im wesentlichen ein hohlzylindrisches Rohr mit zwei offenen Enden 12, 24, in dem ein Öffnungsabschnitt 14, ein erster Hohlraumabschnitt 16 und eine Durchgangsöffnung 18 zu einem zweiten Hohlraumabschnitt 20 angeordnet sind. In einem Teil des zweiten Hohlraumabschnittes 20, der dem offenen Ende 24 benachbart liegt, ist ein Innengewinde 22 vorgesehen. Die Außenkontur der Griffhülse 10 ist im wesentlichen durch ergonomische Gesichtspunkte bestimmt. Als zweckmäßig erwiesen hat sich eine hexagonalsymmetrische Gestaltung, wie sie durch die Draufsicht der Figur 2 angedeutet wird. An der Außenseite der Griffhülse 10 sind Haltemulden, beispielsweise bei 26, und Fingermulden, beispielsweise bei 28, vorgesehen. Der vordere Teil 30 der Griffhülse 10 ist in ein Gewinde 32 des ersten Hohlraumabschnittes 16 einschraubbar.

In die Griffhülse 10 ist ein Schlagfedermechanismus eingesetzt.

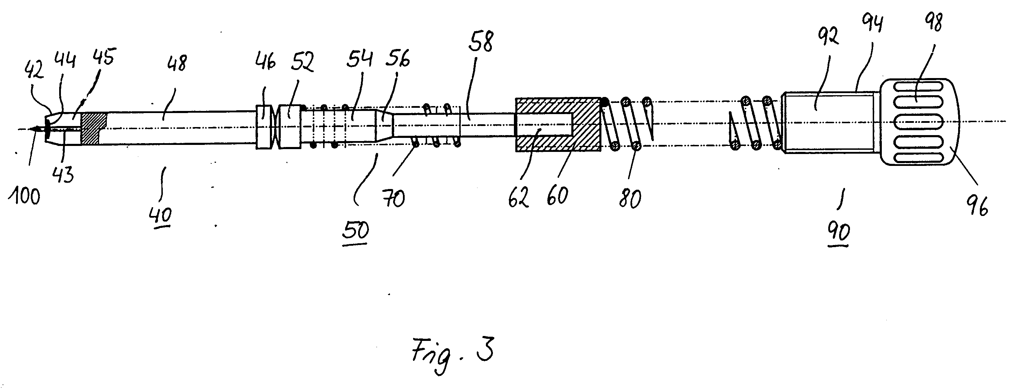

In einer ersten Ausführungsform, wie in Figur 3 dargestellt, besteht dieser aus einem Stößel 40, einem Bolzen 50, um den eine Schraubenfeder 70 gelegt ist, einem Kopplungsstück 60, einer zweiten Schraubenfeder 80 und einer Einstellschraube 90. Dabei sind der Stößel 40 und der Bolzen 50 in dem ersten Hohlraumabschnitt 16 zumindest teilweise untergebracht, wobei der Stößel 40 durch den Öffnungsabschnitt 14 aus dem offenen Ende 12 hervorsteht. An diesem freiliegenden Ende des Stößels 40 ist ein Haltemechanismus für einen Nagel 100 angeordnet. Dieser besteht aus Greifbacken 45, beispielsweise vier Stück, die an ihrer der Griffhülse 10 abgewandten Seite eine Abschrägung 42 aufweisen und in ihrer Gesamtheit eine Anlageschulter 44 für den Kopf des Nagels 100 bilden. Die Greifbacken bilden zwischen sich, je nach Größe des Nagelkopfes, einen mehr oder weniger weiten Kreuzschlitz-Spalt 43. An diesen Haltemechanismus schließt sich ein Schaft 48 an, der in einem Anlageabschnitt 46 endet, dessen Außenabmessungen größer gewählt sind als der Durchmesser des Schaftes. Der Anlageabschnitt 46 definiert einerseits eine Anlageschulter zu einer schrägverlaufenden Ringschulter 15 zwischen dem Öffnungsabschnitt 14 und dem ersten Hohlraumabschnitt 16. Andererseits liegt an den Anlageabschnitt 46, der eine gewölbte Kontaktfläche hat, ein ähnlich ausgebildeter Anlageabschnitt 52 des Bolzens 50 an. Die gewölbten Kontaktflächen der beiden Anlageabschnitte 46, 52 lassen zu, daß die Achsen von Stößel und Bolzen gegeneinander verkippt werden können. Da der Stößel 40 durch den Öffnungsabschnitt 14 in der Griffhülse 10 geführt ist, wobei seine Achse mit der Achse der Griffhülse 10 ausgerichtet ist, bedeutet dies, daß die Achse des Bolzens 50 gegenüber der Achse der Griffhülse 10 verkippt werden kann. Dieses Verkippen wird mit Hilfe der Schraubenfeder 70 erzwungen, die um den Bolzen 50 gelegt ist, wobei sie an dem an den Anlageabschnitt 52 angrenzenden ersten Bolzenabschnitt 54 anliegt. Eine konisch sich verjüngende Zentrierfläche 56 bildet den Übergang zu dem Bolzenabschnitt 58, der demzufolge einen geringeren Durchmesser hat als der Bolzenabschnitt 54. An diesem Bolzenabschnitt 58 liegt die Schraubenfeder 70 nicht an. Das nicht von der Schraubenfeder 70 umgebene Ende des Bolzenabschnittes 58 ragt durch die Durchgangsöffnung 18. In dem zweiten Hohlabschnitt 20 der Griffhülse 10 liegt das Kopplungsstück 60 an der Konusfläche 19, wobei das Blindloch 62 der Durchgangsöffnung 18 zugewandt ist. An der gegenüberliegenden Seite des Kopplungsstückes 60 liegt die Schraubenfeder 80 an, die sich wiederum an dem Schaft 92 der Gewindeschraube 90 abstützt. Durch Drehen der Gewindeschraube 90 am Kopf 96, der zur leichteren Handhabbarkeit mit Griffrillen 98 versehen ist, wird das Gewinde 94 mehr oder weniger in das Innengewinde 22 der Griffhülse 10 eingeschraubt, so daß die Schraubenfeder 80 mehr oder weniger vorgespannt werden kann.

Beim Spannen des Systems wird die Stößel-Bolzen-Anordnung 40, 50 weiter in die Griffhülse 10 hineingeschoben. Da die Schraubenfeder 70 gegenüber der Achse der Griffhülse 10 versetzt ist, sitzt der zweite Bolzenabschnitt 58, dessen Achse durch die Schraubenfeder 70 ebenfalls in eine versetzte Lage gezwungen wird, auf dem Rand des Blindloches 62 auf. Dadurch wird das Kopplungsstück 60 mitgeschoben und dabei die Schraubenfeder 80 weiter zusammengedrückt. Wenn so weit zurückgeschoben ist, daß die Zentrierfläche 56 in den Bereich der Konusfläche 17 kommt, die den ersten Hohlabschnitt 16 und die Durchgangsbohrung 18 verbindet, wird der Bolzen 50 zwangszentriert, so daß sein Abschnitt 58 plötzlich in das Blindloch 62 springt. Sobald der zweite Bolzenabschnitt 58 auf dem Boden des Blindloches 62 aufsitzt, wird, ausgelöst durch die Federkraft der Schraubenfeder 80, der Schlag in Richtung auf den Stößel 40 und den eingesetzten Nagel 100 ausgeführt.

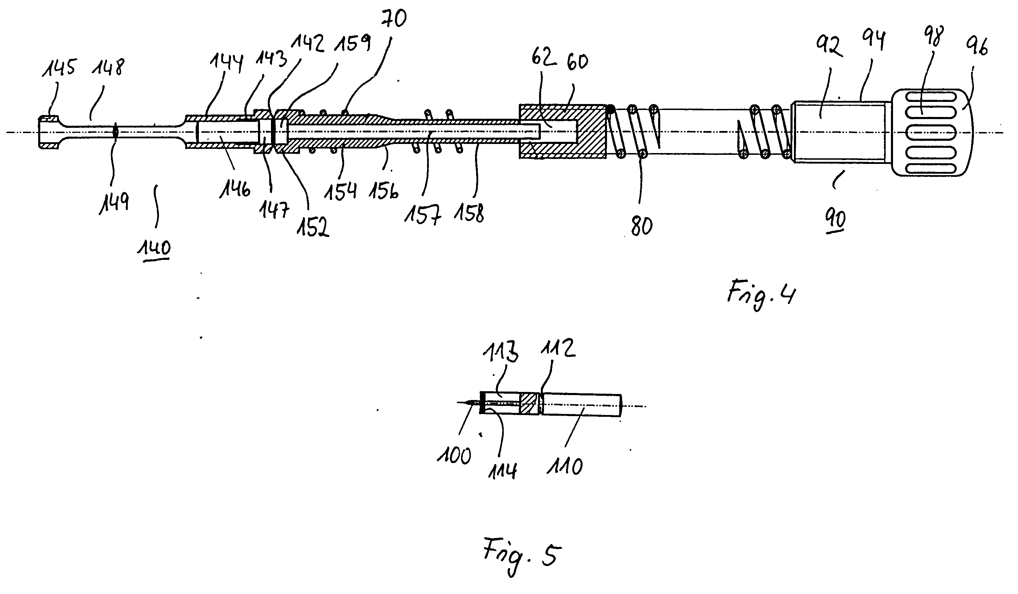

Figur 4 zeigt eine zweite Ausführungsform des Schlagfedermechanismus zur Verwendung bei der vorliegenden Erfindung. Der Stößel 140 ist hier als Hohlzylinder ausgebildet, in den ein Kopplungsbolzen 146 in das Ende eingelegt ist, das dem Bolzen 150 zugewandt ist. Ein Nagelhalter 110, der in Figur 5 dargestellt ist, wird weiter in den Stößel 140 eingesetzt, so daß er an dem Kopplungsbolzen 146 anliegt. Dabei greift eine Rastnut 112 in eine entsprechende Rastrippe 149, die am Innenumfang des Stößels 140 an entsprechender Stelle vorgesehen ist. Durch Sichtfenster 148, die in die Wand des Stößels 140 eingearbeitet sind, kann festgestellt werden, ob der Nagelhalter 110 korrekt in den Stößel 140 eingelegt ist. Ein Nagel 100 wird in dem Nagelhalter 110 wieder durch den Haltemechanismus gehalten, der zuvor beschrieben ist, also durch Greifbacken 113, die den Kopf des Nagels 100 an einer Anlageschulter 114 halten. Das freiliegende Ende des Nagelhalters 110 ragt aus dem Stößel 140 im Endbereich 144 hervor. Der Kopplungsbolzen 146 weist an dem dem Bolzen 150 zugewandten Ende einen etwas vergrößerten Kopf 147 auf, der in einer vergrößerten Innenausnehmung des Stößels 140 gleiten kann und in seiner axialen Bewegung durch diese begrenzt ist. Der Bolzen 150 ist ebenfalls als Hohlbolzen ausgebildet, wobei die äußeren Konturen, also insbesondere der erste Bolzenabschnitt 154, die Zentrierfläche 156 und der zweite Bolzenabschnitt 158 wie beim ersten Ausführungsbeispiel der Figur 3 dimensioniert sind. Auch die Schraubenfeder 70 ist in derselben Weise angeordnet, ihre Achse ist insbesondere zur Achse der Griffhülse 10 versetzt. Im Bolzen 150 ist ein Innenbolzen 157 untergebracht, dessen Kopf 159 auf einem Anlagerand 153 im Bolzen 150 aufsitzt. Der Innenbolzen 157 kann sich also nur in Richtung des Stößels 140 bewegen. Der Innenbolzen 157 ragt weiter über den zweiten Bolzenabschnitt 158 hinaus und in das Blindloch 62 des Kopplungsstückes 60 hinein. Ansonsten entspricht die Anordnung des Stößels 140, des Bolzens 150, des Kopplungsstücks 60, der Schraubenfeder 80 und der Einstellschraube 90 in der Griffhülse 10 (Figur 1) der der ersten Ausführungsform.

Der grundsätzliche Unterschied zu dem Schlagfedersystem der Figur 3 besteht darin, daß beim Spannen des Systems die Kraft nicht über den Nagel 100 eingeleitet wird, sondern über den hohl ausgebildeten Stößel 140. Dadurch wird verhindert, daß der Nagel 100 verkantet bzw. vorzeitig ausfällt. Weiter wird die Schlagbewegung dann ausgelöst, wenn das in das Blindloch ragende Ende des Innenbolzens 157 auf dem Boden des Blindloches aufsitzt.

Die in der vorstehenden Beschreibung, in der Zeichnung sowie in den Ansprüchen offenbarten Merkmale der Erfindung können sowohl einzeln als auch in beliebiger Kombination für die Verwirklichung der Erfindung wesentlich sein.

### BEZUGSZEICHENLISTE

- 10: Griffhülse
- 12: Erstes offenes Ende
- 14: Öffnungsabschnitt
- 15: Konischer Abschnitt
- 16: Erster Hohlabschnitt
- 17: Konusfläche
- 18: Durchgangsöffnung
- 19: Konusfläche
- 20: Zweiter Hohlabschnitt
- 22: Innengewinde
- 24: Zweites offenes Ende
- 26: Haltemulden
- 28: Fingermulden
- 30: vorderer Teil der Griffhülse 10
- 32: Gewinde
- 40: Stößel
- 42: Abschrägung
- 43: Kreuzschlitz-Spalt
- 44: Anlageschulter
- 45: Greifbacke
- 46: Anlageabschnitt
- 48: Schaft
- 50: Bolzen
- 52: Anlageabschnitt
- 54: Erster Bolzenabschnitt
- 56: Zentrierfläche
- 58: Zweiter Bolzenabschnitt
- 60: Kopplungsstück
- 62: Blindloch
- 70: Schraubenfeder
- 80: Schraubenfeder
- 90: Einstellschraube
- 92: Schaft
- 94: Gewinde
- 96: Kopf
- 98: Griffrillen
- 100: Nagel
- 110: Nagelhalter
- 112: Rastkerbe
- 113: Greifbacken
- 114: Anlageschulter
- 140: Stößel
- 142: Anlageabschnitt
- 143: Ausnehmung
- 144: Zylinderwand
- 145: Freier Endabschnitt des Stößels
- 146: Kopplungsbolzen
- 147: Kopf
- 148: Sichtfenster
- 149: Rastrippe
- 150: Bolzen
- 152: Anlageabschnitt
- 153: Anlagerand
- 154: Erster Bolzenabschnitt
- 156: Zentrierfläche
- 157: Innenbolzen
- 158: Zweiter Bolzenabschnitt
- 159: Kopf des Innenbolzens

## Patentansprüche

1. Setzwerkzeug für Nägel, mit
- einer an beiden Enden (12, 24) offenen hohlzylindrischen Griffhülse (10), bei der ein erster Hohlabschnitt (16) mit einem zweiten Hohlabschnitt (20) durch eine Durchgangsöffnung (18) verbunden ist, in die folgende Komponenten eingelegt sind:
- ein Stößel (40; 140), an dem ein Nagel (100) direkt oder indirekt lösbar und aus der Griffhülse (10) freiliegend zu halten ist,
- ein Bolzen (50; 150), der von einer Schraubenfeder (70) umgeben ist, deren Achse aus der Achse der Griffhülse (10) versetzt ist, und der mit dem Anlageabschnitt eines ersten Bolzenabschnitts (54; 154) an dem Stößel (40, 140) anliegt und bei dem im Übergangsbereich von dem ersten Bolzenabschnitt (54; 154) zu einem zweiten Bolzenabschnitt (58; 158) eine Zentrierfläche (56; 156) vorgesehen ist,
- ein zylindrisches Kopplungsstück (60), das an seiner dem Bolzen (50; 150) zugewandten Seite mit einem Blindloch (62) versehen ist, in das der zweite Bolzenabschnitt (58; 158) des Bolzens (50; 150) zumindest teilweise eintauchen kann und dessen Durchmesser kleiner ist als der Durchmesser der Durchgangsöffnung (18);
- eine zweite Schraubenfeder (80),
- eine Einstellvorrichtung (90), die verstellbar in das zweite offene Ende (24) der Griffhülse (10) eingesetzt ist,
wobei die zweite Schraubenfeder (80) einerseits an dem Kopplungsstück (60) und andererseits an der Einstellvorrichtung (90) anliegt und wobei der Stößel (40; 140) und der Bolzen (50; 150) mit der ihn umgebenden Schraubenfeder (70) zumindest teilweise im ersten Hohlabschnitt (10) und das Kopplungsstück (60), die zweite Schraubenfeder (80) und die Einstellvorrichtung (90) im zweiten Hohlabschnitt (20) untergebracht sind.

2. Setzwerkzeug nach Anspruch 1, dadurch gekennzeichnet, daß der Anlageabschnitt (52) des Bolzens (50) an den Stößel (40) und/oder der Anlageabschnitt (46) des Stößels (40) an den Bolzen (50) an ihrem Kontaktbereich gewölbt sind.

3. Setzwerkzeug nach Anspruch 1, dadurch gekennzeichnet, daß das freie Ende (42) des Stößels (40) als Halterung (42, 44) für den Kopf eines Nagels (100) ausgebildet ist.

4. Setzwerkzeug nach Anspruch 1, dadurch gekennzeichnet, daß der Stößel (140) und der Bolzen (150) hohlzylindrisch ausgebildet sind, wobei in den Bolzen (150) ein Innenbolzen (157) untergebracht ist, dessen Länge größer ist als die Länge des Bolzens, und der direkt oder indirekt auf einen Nagelhalter (110) wirkt, welcher in den Stößel (140) eingesetzt ist.

5. Setzwerkzeug nach Anspruch 4, dadurch gekennzeichnet, daß in dem Stößel (140) ein Kopplungsbolzen (146) untergebracht ist.

6. Setzwerkzeug nach Anspruch 4 oder 5, dadurch gekennzeichnet, daß der Stößel (140) Sichtfenster (148) aufweist.

7. Setzwerkzeug nach einem der Ansprüche 4 bis 6, dadurch gekennzeichnet, daß der Stößel (140) eine Rasteinrichtung (149) zum Eingriff mit dem Nagelhalter (110) aufweist.

8. Setzwerkzeug nach einem der Ansprüche 1 bis 7, dadurch gekennzeichnet, daß die Einstellvorrichtung (90) eine Einstellschraube ist, deren Gewindeabschnitt (92, 94) in ein in der Griffhülse (10) vorgesehenes Innengewinde (22) eingreift.
